# EUROPEAN PATENT APPLICATION

(11) **EP 4 678 734 A1**
(43) Date of publication of application: **14.01.2026**
(21) Application number: 25154284.1
(22) Date of filing: 27.01.2025
(51) Int. Cl.: C12N 1/20, A01N 63/20, C05F 11/08, C12N 11/04

(54) **BIOINOCULATE AND THE METHOD OF OBTAINING BIOINOCULATE**

(30) Priority: 12.07.2024 PL 44922424
(71) Applicant: Uniwersytet Mikolaja Kopernika w Toruniu, 87-100 Torun (PL)
(72) Inventor: Hrynkiewicz, Katarzyna, 87-148 Papowo Torunskie (PL); Deja-Sikora, Edyta, 87-100 Torun (PL); Thiem, Dominika, 87-123 Brzozówka (PL); Wegrzyn, Ewa, 61-619 Poznan (PL)

(57) **Abstract**

The subject of the invention is a bioinoculum containing a strain of endophytic bacteria and biochar for use as an agricultural biopreparation. A bioinoculum containing an endophytic bacterial strain and biochar is characterised by that the bacterial strain belongs to the genus *Labedella.*

The subject of the invention is also a method of obtaining a bioinoculum containing an endophytic bacterial strain and biochar for use as an agricultural biopreparation. The method of obtaining bioinoculum according to the invention above is characterised by that the initial liquid culture of bacteria of the genus *Labedella* is transferred to a sterile liquid medium in the ratio of 1 ml of bacterial culture to 100 ml of medium and incubated on a shaker at 120-145 rpm, at a temperature in the range 24-26°C until the bacteria reach a logarithmic growth phase, the biochar is then sieved through a sieve with a mesh size of 0.75 mm or less, whereupon the biochar is mixed with the bacterial suspension at a ratio of 5:100 w/v under sterile conditions and shaken for 2 h on a shaker at 120-145 rpm, at a temperature in the range 24-26°C, then 2% w/v of sodium alginate is added under sterile conditions and shaking is continued for 1 h until a homogeneous mixture is obtained, the resulting suspension is dropped into the mixed and sterilised 2% w/v calcium chloride solution on a magnetic stirrer from a height of 15 cm to 20 cm, then the resulting granules are left in the calcium chloride solution at 4°C for 24 h to harden, and then the collected granules are washed with sterile water and lyophilised for 12-20 h.

## Description

The subject of the invention is a bioinoculum containing a strain of endophytic bacteria and biochar for use as an agricultural biopreparation. The subject of the invention is also a method of obtaining a bioinoculum containing an endophytic bacterial strain and biochar for use as an agricultural biopreparation.

Endophytic bacteria are microorganisms that live inside plant tissues without causing any diseases.These are bacteria that inhabit the plant interiors, such as leaves, stems, roots or seeds, and maintain a symbiotic, often mutually beneficial relationship with plants. They provide plants with various benefits, such as increased stress resistance, improved nutrient uptake and growth hormone production. When used as biological fertilizers, they increase the efficiency of nutrients absorption by plants.

Biochar is used as a carrier for microorganisms for agricultural and environmental purposes, such as soil bioremediation. The rough and porous surface of biochar provides microorganisms with space for colonisation.

A microbial inoculum containing *Pseudomonas fluorescens, Enterobacter carcinomatosis* and *Azotobacter bailii* and biochar is known from the Chinese patent CN117363527A. In this invention, pomegranate peel biochar is incubated with microorganisms in order to adsorb and immobilise microorganisms, so that the protective effect of the biochar on the microorganisms in the soil is enhanced. The method of obtaining the inoculum consists of culturing three strains of bacteria (*Pseudomonas fluorescens, Enterobacter carcinomatosis* and *Azotobacter bailii*), combining them in equal proportions and adding sterilised biochar at a ratio of 100 g of biochar to 1L of fermentation medium and growing them together at a temperature of 28-30 Celsius degrees on a shaker, at 150-200 rpm for 28-30 days, followed by drying them to obtain the microbial agent.

A microbial inoculum based on a cornstraw biochar containing *Pantoea* ZHJ28 strain and a method of preparing the biochar-based microbial inoculum are known from the Chinese patent description CN117487715A. The ratio of biochar to microbial agent is from 0.5-1 g to 2.5 ml, and the microbial cell density is from 1×10^8 to 1×10^9 cfu/g. The method of preparing the biochar-based microbial inoculum includes culturing the *Pantoea* ZHJ28 strain, preparing the biochar, and then mixing and co-digesting the biochar and microbial agent. A biochar-based microbial inoculum can be used to remediate soil contaminated with microplastics and to support the crop growth, especially peanuts. Experiments have shown that it improves peanut growth parameters and photosynthesis compared to the control.

The purpose of the invention is to provide an environmentally natural bioinoculum based on an endophytic bacterium in combination with biochar and to develop a novel method of formulating such a bioinoculum.

The essence of the invention is a bioinoculum containing a strain of an endophytic bacterium and a biochar characterised in that the bacterial strain belongs to the genus *Labedella. Labedella* bacteria participate in the biodegradation of organic pollutants in the soil, helping to clean the agricultural environment of toxins and contaminants. *Labedella* is also involved in the detoxification of heavy metals. These bacteria interact with fertilisers to improve their effectiveness, to reduce the use of chemical fertilisers and to mitigate the effects of environmental stresses on host plants.

It is beneficial if the bioinoculum contains biochar and the *Labedella* bacterial strain in a 5:100 w/v ratio. This weight-volume ratio ensures the adequate bacterial density.

Preferably, the particle size of the biochar is at most 0.75 mm, preferably is in the range of 0.50 mm - 0.75 mm. Such a fragmentation of biochar ensures that it combines more smoothly with the bacterial suspension and achieves homogeneity.

Preferably, the bioinoculum is in a granular form.

The bioinoculum according to the invention improves the growth and development of plants and increases yield and plant tolerance to adverse environmental conditions. The developed bioinoculum presents an innovative approach combining two environmentally friendly elements: plant growth-promoting *Labedella* bacteria and biochar.

The essence of the invention is also a method of obtaining bioinoculum according to the invention above, characterised in that the initial liquid culture of *Labedella* bacteria is transferred to a sterile liquid medium at a ratio of 1 ml of bacterial culture to 100 ml of medium and incubated on a shaker at 120-145 rpm, at a temperature in the range 24-26°C, until the bacteria reach a logarithmic growth phase. Then, the biochar is sieved through a sieve with meshes of at most 0.75 mm, so that it can easily combine with the bacterial suspension. The biochar is then mixed with the bacterial suspension in a ratio of 5:100 w/v under sterile conditions and shaken for 2 h on a shaker at 120-145 rpm, at a temperature in the range of 24-26°C. Then, 2% w/v of sodium alginate is added under sterile conditions and shaking is continued for 1h until a homogeneous mixture is obtained. The resulting suspension is dropped into a mixed and sterilised 2% w/v calcium chloride solution on a magnetic stirrer from a height of 15 cm to 20 cm. The resulting granules are left in calcium chloride solution for 24 h at 4°C to harden. The collected granules are washed with sterile water and lyophilised for 12-20 h. The addition of sodium alginate stabilises the bacterial suspension, while adding drops into the calcium chloride solution and the low dispersion of the biochar allows the formation of uniform granules.

Preferably, the initial liquid culture of *Labedella* bacteria has an optical density of 1. At this value, the bacterial cell count is 3 × 10^8 CFU/ml. Maintaining OD = 1 promotes the efficient production of bioinoculum, as it ensures the adequate quantity of live and active bacterial cells that are necessary for the successful production of bioinoculum.

Preferably, R2A medium is used as a liquid medium. R2A medium reduces bacterial stress and ensures optimal microbial growth.

The method of producing bioinoculum according to the invention makes it possible to obtain stable granules that can be used as a preparation enriching the soil in which crops used in agriculture are grown. The result is a bioinoculum that increases crop yields and improves their tolerance to adverse environmental conditions. The developed bioinoculum is applicable under adverse environmental conditions (abiotic stress), e.g. drought, and for various plants. The method for the preparation is universal. A plant growth-promoting *Labedella* bacterium and biochar were used in one bioinoculum, achieving a double benefit for plant growth and development.

The invention is presented below in embodiments, which are illustrative and do not in any way limit the claimed invention.

### Example I

The bioinoculum according to the invention is a granulate containing the *Labedella endophytica* bacterial strain and biochar containing 5 g of biochar in 100 ml of bacterial suspension of the *Labedella endophytica* strain. Bioinoculum contains biochar particles with a size of 0.50 mm-0.75 mm.

### Example II

The bioinoculum according to the invention is a granulate containing the *Labedella endophytica* bacterial strain and biochar with a content of 10 g of biochar in 200 ml of bacterial suspension of the *Labedella endophytica* bacterial strain. Bioinoculum contains biochar particles with a size of 0.30 mm-0.55 mm.

### Example III

The method of obtaining bioinoculum according to the invention includes the following steps:

### Preparation of Labedella endophytica bacterial strain suspension:

1 ml of a liquid overnight bacterial culture of *Labedella endophytica* with an optical density value of OD = 1 is transferred to 100 ml of sterile liquid R2A medium. The optical density (OD) is measured on a densitometer. An OD = 1 value indicates bacterial cells at 3 × 10^8 CFU/ml. The bacterial suspension is incubated for 18 h on a shaker at 120 rpm at 25°C until the bacteria reach a logarithmic growth phase.

### Preparation of biochar:

The biochar is sieved through a sieve with meshes ≤ 0.75 mm. A biochar fraction of 0.50 mm - 0.75 mm is used.

### Preparation of bioinoculum:

Biochar in an amount of 5 g is mixed with 100 ml of a bacterial suspension of the *Labedella endophytica* strain under sterile conditions and shaken for 2 h on a shaker at 120 rpm at 25°C. Then, 2 g of sodium alginate is added under sterile conditions and shaking is continued for 1 h until a homogeneous mixture is obtained. The resulting suspension is dropped into a well-mixed and sterilised 2% (w/v) calcium chloride solution on a magnetic stirrer from a height of 15 cm. The resulting granules are left in calcium chloride solution for 24 h at 4°C to harden. Then, the granules are collected and flushed with sterile water. The granules are then freeze-dried for 12 h. The bioinoculum is stored at a temperature of 4°C until use.

### Example IV

The method of obtaining bioinoculum according to the invention includes the following steps:

### Preparation of Labedella endophytica bacterial strain suspension:

3 ml of liquid bacterial culture of *Labedella endophytica* with an optical density value of OD = 1 is transferred into 300 ml of sterile liquid R2A medium. The bacterial suspension is incubated at 24°C for 22 h on a shaker at 145 rpm until the bacteria reach a logarithmic growth phase.

### Preparation of biochar:

The biochar is sieved through a sieve with meshes ≤ 0.50 mm. A biochar fraction of 0.35 mm -0.50 mm is used.

### Preparation of bioinoculum:

20 g of biochar is mixed with 400 ml of a bacterial suspension of the *Labedella endophytica* strain under sterile conditions and shaken for 2 h on a shaker at 145 rpm at 24°C. Then, 8 g of sodium alginate is added under sterile conditions and shaking is continued for 1 h until a homogeneous mixture is obtained. The resulting suspension is dropped into a well-mixed and sterilised 2% (w/v) calcium chloride solution on a magnetic stirrer from a height of 20 cm. The resulting granules are left in calcium chloride solution for 24 h at 4°C to harden. Then, the granules are collected and flushed with sterile water. The granules are then freeze-dried for 15 h. Bioinoculum is stored at 4°C until use.

### Example V

The method of obtaining bioinoculum according to the invention includes the following steps:

### Preparation of Labedella endophytica bacterial strain suspension:

2 ml of liquid, overnight bacterial culture of *Labedella endophytica* with an optical density value of OD = 1 is transferred into 200 ml of sterile liquid R2A medium. The bacterial suspension is incubated for 15 h on a shaker at 130 rpm at 26°C until the bacteria reach a logarithmic growth phase.

### Preparation of biochar:

The biochar is sieved through a sieve with meshes ≤ 0.70 mm. A biochar fraction of 0.50 mm - 0.70 mm is used.

### Preparation of bioinoculum:

Biochar in an amount of 7 g is mixed with 140 ml of a bacterial suspension of the *Labedella endophytica* strain under sterile conditions and shaken for 2 h on a shaker at 120 rpm at 26°C. Then, 2.8 g of sodium alginate is added under sterile conditions and shaking is continued for 1 h until a homogeneous mixture is obtained. The resulting suspension is dropped into a well-mixed and sterilised 2% (w/v) calcium chloride solution on a magnetic stirrer from a height of 18 cm. The resulting granules are left in calcium chloride solution at 4°C for 24 h to harden. Then, the granules are collected and flushed with sterile water. The granules are then freeze-dried for 20 h. Bioinoculum is stored at 4°C until use.

The bioinoculum obtained by the method according to the invention was introduced into the soil immediately after sowing the seeds of a plant belonging to Poaceae family. The crop was grown for 8 weeks under phytotron conditions. After this time, plant growth parameters were determined: the length and weight of stems and roots.
Three variants were prepared. In the first variant, only seeds were sown; in the second and the third variants, seeds were sown together with granulate. The experiment was conducted in a phytotron for eight weeks. Watering was performed regularly. After this time, 40 plants from each variant were checked and the length of the stem and root, as well as the fresh and dry weight of the stem and root, were measured. The values obtained were statistically analysed, and the Shapiro-Wilk test, Levene's test, Kruskal-Wallis rank ANOVA and Dunn's post-hoc test were performed. The results obtained are shown in Table 1.

**Table. 1. Mean values from measurements of stem and root length, stem and root fresh weight and stem and root dry weight.**

| | **stem length (cm)** | **root length (cm)** | **fresh stem weight (mg)** | **fresh root weight (mg)** | **dry stem weight (mg)** | **dry root weight (mg)** |
|---|---|---|---|---|---|---|
| **G2-Ctrl** | 48.26 | 13.05 | 268.44 | 144.38 | 61.86 | 30.99 |
| **G2-N2** | 44.69 | 14.19 | 218.26 | 173.92 | 79.91 | 47.73 |
| **G2-N2-B1** | 51.07 | 17.12 | 365.69 | 285.77 | 95.36 | 45.59 |

| | | | | | | |
|---|---|---|---|---|---|---|
| G2-Ctrl - as control, non-inoculated plants G2-N2 - granulate without bacteria, with water G2-N2-B1 - bacteria granulate with biochar | | | | | | |

The following parameters were statistically significantly different: root length, stem and root fresh weight, stem and root dry weight. The figure shows bar chart with the results of the individual parameter measurements for 3 variants: non-inoculated plants, for plants with the addition of granulate without bacteria, and for plants with the addition of bioinoculum according to the invention. In the charts, asterisks indicate variants that are statistically significantly different compared to the control.

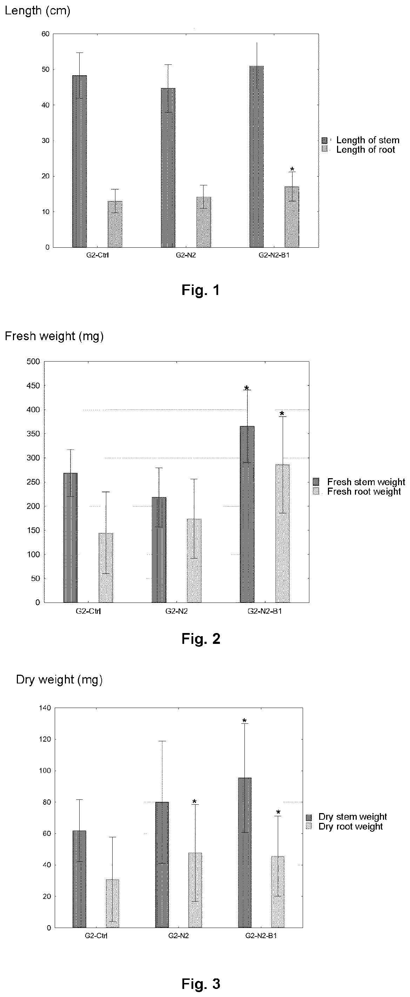

Fig. 1 shows the average stem and root length for each variant. The variant with the bacteria is statistically significantly different compared to the control in case of the root length.

Fig. 2 shows the average weight of the fresh stem and root in the different variants. In both cases, the variant with the bacteria is statistically significantly different compared to the control.

Fig. 3 shows the average weight of the dry stem and root in the different variants. Both the no-bacteria and bacteria variants are statistically significantly different compared to the control in case of the dry root weight. The bacteria variant is also statistically significantly different compared to the control in case of the dry stem weight.

The roots showed a significantly higher length by 31% compared to the control variant without bioinoculum, significantly higher fresh weight by 98% compared to the control variant without bioinoculum, and a significantly higher dry weight by 47% compared to the control variant without bioinoculum. The stems showed a significantly higher fresh weight by 36% compared to the control variant without bioinoculum, and a significantly higher dry weight by 54% compared to the control variant without bioinoculum.

## Claims

1. Bioinoculum containing an endophytic bacterial strain and biochar **characterised in that** the bacterial strain belongs to the genus *Labedella.*

2. Bioinoculum according to claim 1 **characterised in that** it contains biochar and a strain of bacteria of the genus *Labedella* in a ratio of 5:100 w/v.

3. Bioinoculum according to claim 1 or 2 **characterised in that** the particle size of the biochar is at most 0.75 mm, preferably in the range 0.50 mm - 0.75 mm.

4. Bioinoculum according to any of the claims from 1 to 3 **characterised in that** it is in granulate form.

5. Method of obtaining bioinoculum according to any of the claims from 1 to 4 **characterised in that** an initial liquid culture of bacteria of the genus *Labedella* is transferred to a sterile liquid medium in the ratio of 1 ml of bacterial culture to 100 ml of medium and incubated on a shaker at 120-145 rpm, at a temperature in the range 24-26°C until the bacteria reach a logarithmic growth phase,
then, the biochar is sieved through a sieve with a mesh of at most 0.75 mm, whereupon the biochar is mixed with the bacterial suspension in a ratio of 5:100 w/v under sterile conditions and shaken for 2 h on a shaker at 120-145 rpm, at a temperature in the range 24-26°C, then 2% w/v of sodium alginate is added under sterile conditions and shaking is continued for 1 h until a homogeneous mixture is obtained,
the resulting suspension is dropped into a mixed and sterilised 2% w/v calcium chloride solution on a magnetic stirrer from a height of 15 cm to 20 cm,
the resulting granules are left in a calcium chloride solution for 24 h at 4°C to harden,
the collected granules are washed with sterile water and lyophilised for 12-20 h.

6. Method of obtaining bioinoculum according to claim 5 **characterised in that** the liquid initial culture of bacteria of the genus *Labedella* has an optical density of 1.

7. Method of obtaining bioinoculum according to claim 5 or 6 **characterised in that** R2A medium is used as a liquid medium.
